# EUROPÄISCHE PATENTSCHRIFT

(11) **EP 1 415 621 B1**
(45) Veröffentlichungstag und Bekanntmachung des Hinweises auf die Patenterteilung: **14.09.2016**
(21) Anmeldenummer: 03010285.9
(22) Anmeldetag: 07.05.2003
(51) Int. Cl.: A61F 2/40

(54) **Schultergelenkprothese**
Shoulder prothesis
Prothèse de l'épaule

(30) Priorität: 21.08.2002 EP 02018730
(43) Veröffentlichungstag der Anmeldung: 06.05.2004
(73) Patentinhaber: Zimmer GmbH, 8404 Winterthur (CH)
(72) Erfinder: Rauscher, Markus, 6319 Allenwinden (CH); Wendt, Peter, 8542 Wiesendangen (CH)
(74) Vertreter: Manitz, Finsterwald & Partner GbR

(56) Entgegenhaltungen:
- EP-A- 0 821 924
- EP-A- 0 845 250
- EP-A- 1 048 274
- EP-A- 1 059 071
- EP-A- 1 125 565
- WO-A-00/18335
- WO-A-99/37254
- US-A- 3 228 393
- US-A1- 2002 099 445

## Beschreibung

Die Erfindung betrifft eine Schultergelenkprothese mit zwei zusammenwirkenden Lagerkörpern, einem Schaft und einer Kupplung zum Verbinden des Schaftes mit einem der Lagerkörper.

Eine derartige Prothese ist beispielsweise aus den europäischen Patentanmeldungen EP 01 811 120.3 und EP 02 018 730.8 bekannt.

US 2002/099445 A1 offenbart eine Prothese gemäß dem Oberbegriff des Anspruchs 1.

Bei einem derartigen flexiblen System ist der in den Oberarm einzusetzende Schaft nicht fest mit dem Lagerkörper verbunden, sondern es können für den jeweiligen Fall passende, aus einem Baukastensystem auswählbare Lagerkörper über die Kupplung mit dem Schaft verbunden werden, wobei es außerdem möglich ist, unterschiedliche Stellungen des Lagerkörpers relativ zum Schaft zu realisieren. Diese Flexibilität erfordert es, den Schaft derart auszubilden, dass im Bereich des Übergangs zwischen Schaft und Lagerkörper ausreichend Platz vorhanden ist, um verschiedene Lagerkörper anzubringen bzw. verschiedene Relativstellungen der Lagerkörper zu ermöglichen. Bei Frakturen mit einer Vielzahl von kleinen Knochenfragmenten kann es zu Problemen beim Zusammenwachsen der einzelnen Trümmerstücke kommen.

Aufgabe der Erfindung ist es, bei einer Schultergelenkprothese der eingangs genannten Art unter Beibehaltung der Flexibilität und ohne Beeinträchtigung der Verbindung zwischen Lagerkörper und Schaft für optimale Ergebnisse auch bei Frakturen mit einer Vielzahl von kleinen Knochenfragmenten zu sorgen.

Die Lösung dieser Aufgabe erfolgt durch die Merkmale des Anspruchs 1 und insbesondere dadurch, dass sich an die Unterseite des mit dem Schaft verbindbaren Lagerkörpers ein Stützkörper anschließt, der im mit dem Schaft verbundenen Zustand mit einer äußeren Stützfläche eine zwischen dem Lagerkörper und dem Schaft vorhandene Lücke zumindest teilweise derart ausfüllt, dass das Zusammenwachsen von Knochenfragmenten begünstigt wird. Der Schaft ist mit einem Stützelement versehen, das seitlich auf dem Schaft aufsitzt und im Bereich des Übergangs vom Schaft in den Stützkörper mit seiner Außenseite die in die Stützfläche des Stützköpers übergehende Mantelfläche des Schaftes bildet.

Durch den Stützkörper wird der Zwischenraum zwischen dem Schaft und dem Lagerkörper überbrückt, wodurch in vorteilhafterweise ein Unterbau geschaffen wird, an welchem sich Knochenfragmente abstützen können, was deren Zusammenwachsen begünstigt.

Die Erfindung ist grundsätzlich unabhängig davon, ob es sich bei dem Lagerkörper um einen Lagerkopf oder um eine Lagerschale handelt.

In einer Weiterbildung der Erfindung ist die Stützfläche des Stützkörpers zumindest bereichsweise konvex ausgeführt.

Ferner kann vorgesehen sein, dass die Mantelfläche des Schaftes und die Stützfläche des Stützkörpers zusammen eine zumindest im Wesentlichen geschlossene, sich an die Unterseite des Lagerkörpers anschließende Fläche bilden.

Die Stützfläche des Stützkörpers kann zumindest näherungsweise tangential an die Mantelfläche des Schaftes anschließen.

Das Heranwachsen von Knochenmaterial wird weiter begünstigt, wenn gemäß einer Ausführungsform der Erfindung die Stützfläche des Stützkörpers strukturiert ist.

Die Stützfläche des Stützkörpers ist insbesondere durchbrechungsfrei ausgebildet.

Der Stützköper kann auswechselbar mit dem Lagerkörper verbunden sein. Hierdurch kann in vorteilhafter Weise eine Modulbauweise realisiert werden, bei der eine Mehrzahl von Lagerkörpern mit einer Mehrzahl von Schutzkörpern kombiniert werden kann, um eine für den jeweiligen Anwendungsfall optimal passende Konfiguration zu bilden.

Des Weiteren kann erfindungsgemäß vorgesehen sein, dass bei mit dem Schaft verbundenem Lagerköper zwischen dem Schaft und dem Stützkörper wenigstens eine Aussparung vorhanden ist, an die ein Werkzeug zum Lösen des Lagerkörpers vom Schaft ansetzbar ist.

Nach einer Ausführungsform kann das Stützelement korb- oder schalenartig ausgebildet sein. Ferner kann das Stützelement fest mit dem Schaft verbunden, beispielsweise verschweißt, sein. Alternativ kann das Stützelement einteilig mit dem Schaft ausgebildet sein.

In einer weiteren Ausgestaltung der Erfindung kann das Stützelement mit Durchbrüchen versehen sein. Ferner kann vorgesehen sein, dass entlang einer Umfangslinie des Stützelementes, die im implantierten Zustand in einer senkrecht zur Schaftachse und in proximaler Richtung in einem Abstand von 8 mm von dem Schnittpunkt zwischen der Schaftachse und der Kopf- bzw. Schalenachse verlaufenden Ebene liegt, der Anteil der aufgrund der Durchbrüche unterbrochenen Abschnitte der Außenfläche des Stützelementes im Bereich von 35 % bis 45 % liegt.

Des Weiteren kann vorgesehen sein, dass bezogen auf die Orientierung des Stützelementes im implantierten Zustand mit senkrecht zur Transversalebene verlaufender Schaftachse das Verhältnis X/Y aus maximalem Außendurchmesser des Stützezelementes in sagittaler Richtung X und maximalem Außendurchmesser des Stützelementes in transversaler Richtung Y im Bereich von 0,85 bis 0,85 liegt.

Die Kupplung kann zur Verbindung mit dem Schaft einen Spannabschnitt umfassen, mit dem durch Einbringen, insbesondere Einschlagen, in eine Kupplungsaufnahme des Schaftes ein fester, insbesondere wieder lösbarer Spannsitz der Kupplung im Schaft herstellbar ist.

Dabei kann vorgesehen sein, dass der Spannabschnitt sich konusartig verjüngt und in eine entsprechend geformte, als Kupplungsaufnahme dienende Gegenform des Schaftes einbringbar ist.

Des Weiteren kann vorgesehen sein, dass der Spannabschnitt im mit dem Schaft verbundenen Zustand einen zu einer Längsachse drehfesten und durch seine Konizität verkeilten Formschluss mit der Gegenform bildet. Hierdurch kann ein selbsthemmender Sitz des Spannabschnitts in der Gegenform realisiert werden.

Der Spannabschnitt kann einen von einer Kreisform abweichenden Querschnitt aufweisen. Vorzugsweise ist der Querschnitt des Spannabschnitts elliptisch.

Dabei kann vorgesehen sein, dass der elliptische Querschnitt des Spannabschnitts und der Gegenform des Schaftes derart in seiner Ebene ausgerichtet ist, dass die große Achse der Ellipse in einer Projektion gegen lateral als Senkrechte erseheint.

Es hat sich gezeigt, dass hierdurch eine optimale Ausnutzung des Materials und ein Maximum an Festigkeit für die Klemmverbindung zwischen clem Spannabschnitt und der Gegenferm erreicht werden kann.

Ferner kann vorgesehen sein, dass der Schaft in der Ebene des elliptischen Querschnitts den Umriss eines mit abgerundeten Ecken versehenen Rechtecks aufweist, dessen langen Seiten parallel zu der großen Achse der Ellipse verlaufen.

Weitere Ausführungsformen der Erfindung sind in den Unteransprüchen, der Beschreibung sowie der Zeichnung angegeben.

Die Erfindung wird im Folgenden beispielhaft unter Bezugnahme auf die Zeichnung beschrieben. Es zeigen:
- Fig. 1: schematisch einen in einem Oberarmknochen implantierten Schaft einer bekannten Schultergelenkprothese,
- Fig. 2: schematisch ein künstliches bekanntes Schultergelenk mit einem als Lagerkopf ausgebildeten Lagerkörper, der einen zu dem Schaft von Fig. 1 passenden konischen Körper aufweist,
- Fig. 3: schem atisch einen Querschnitt durch einen konischen Köper gemäß Fig. 2 mit einem elliptisch verlaufenden Umfang,
- Fig. 4: schematisch einen Schnitt gemäß Fig. 3, an dem eine drehfeste Verkeilung zwischen konischem Körper und Gegenform dargestellt ist,
- Fig. 5: schematisch einen Schnitt durch einen bekannten kon ischen Körper m it einem ursprünglichen Rechteckquerschnitt, an dem nachträglich konische, im Schnitt elliptische Teilflächen passend zu einer Gegenform gemäß Fig. 4 hergestellt wurden,
- Fig. 6: schematisch einen bekannten konischen Körper, bei dem im mittleren Bereich der konischen Fläche eine Unterbrechung eingearbeitet ist,
- Fig. 7: schematisch einen in einem Oberarmknochen implantierten, bekannten Schaft mit einer Gegenform für einen konischen Körper,
- Fig. 8: schematisch einen zum Schaft von Fig. 7 passenden konischen Körper, der mit einem als Kugelschale ausgebildeten Lagerkörper ein künstliches Schultergelenk zu einem Kugelkopf bildet, der über eine Plattform am Schulterknochen befestigt ist,
- Fig. 9: einen Teil einer erfindungsgemäßen Schultergelenkprothese mit einem als Lagerkopf ausgebildeten Lagerkörper im mit einem Schaft verbundenen Zustand,
- Fig. 10: eine andere Ansicht der erfindungsgemäßen Schultergelenkprothese von Fig. 9, Fig. 11 eine weitere Ansicht der erfindungsgemäßen Schultergelenkprothese von Fig. 9, Fig. 12 den mit einem Stützkörper und einem Spannkonus versehenen Lagerkörper der erfindungsgemäßen Schultergelenkprothese von Fig. 9,
- Fig. 13: einen Teil einer anderen Ausführungsform einer erfindungsgemäßen Schultergelenkprothese mit einem als Lagerschale ausgebildeten Lagerkörper,
- Fig. 14: den mit einem Stützkörper und einem Spannkonus versehenen Lagerkörper der erfindungsgemäßen Schultergelenkprothese von Fig. 13,
- Fig. 15: eine Ansicht von oben eines Stützelementes einer Schultergelenkprothese gemäß einer Ausführungsform der Erfindung zur Erläuterung eines Außendurchmesserverhältnisses, und
- Fig. 16a und 16b: verschiedene Ansichten des Stützelementes von Fig. 15.

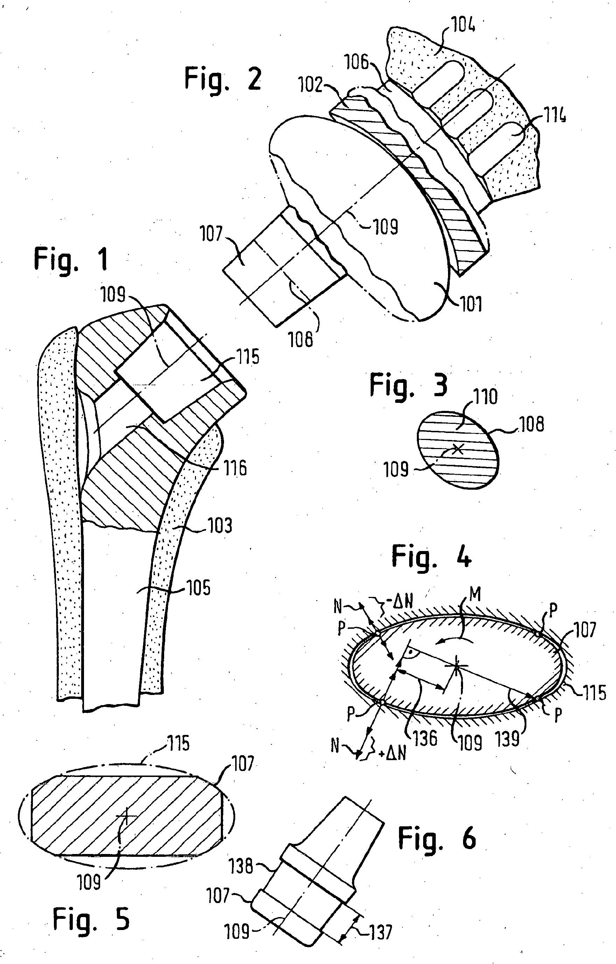

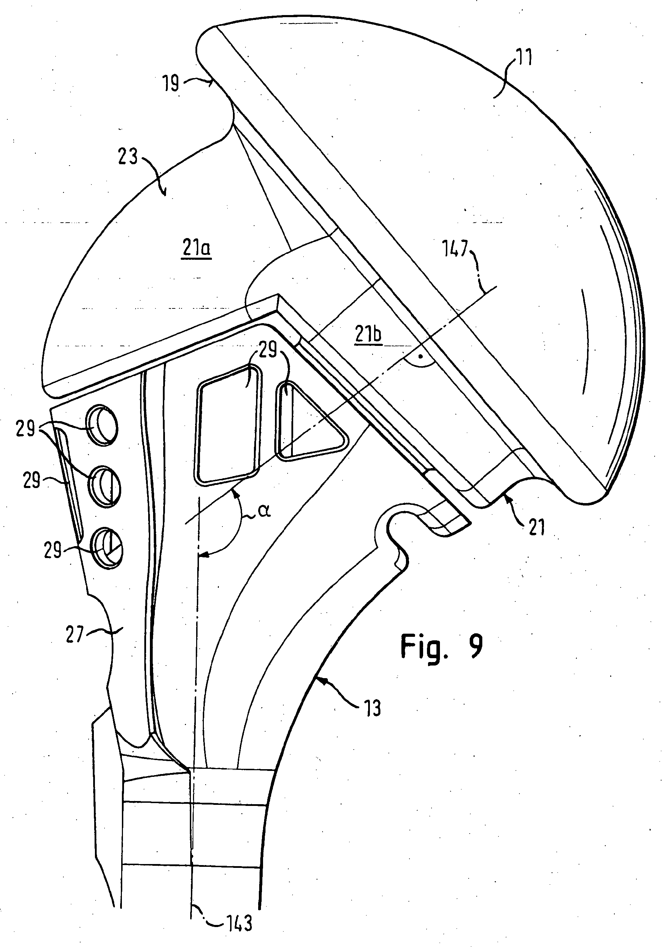

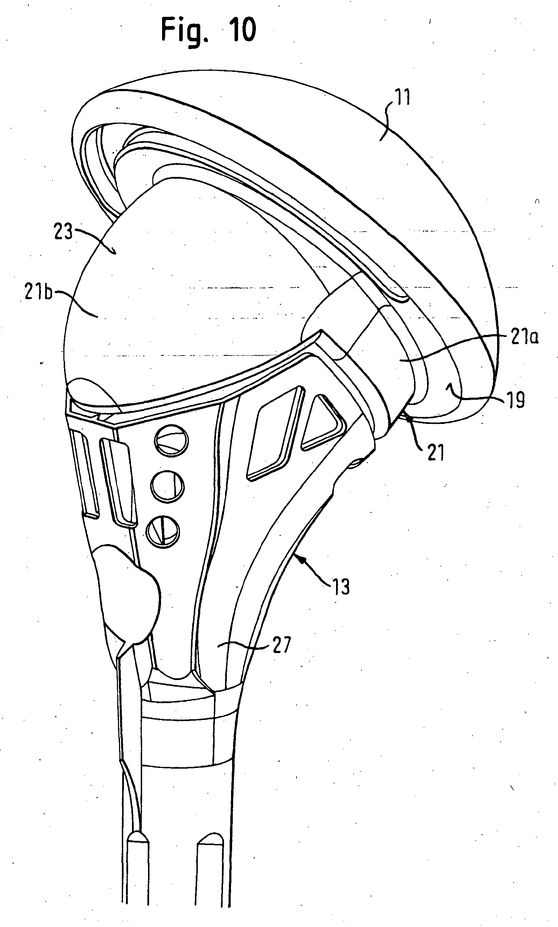

Die Fig. 1 bis 8 zeigen eine bekannten Schultergelenkprothese, deren Merkmale jedoch mit einer Ausnahme, auf die nachstehend eingegangen wird, auch Gegenstand der erfindungsgemäßen Schultergelenkprothese sind oder sein können. Insoweit sind die Fig. 1 bis 8 sowie die dazugehörige Beschreibung als zu der Erfindung gehörend offenbart anzusehen. Die erwähnte Ausnahme besteht darin, dass die Lagerkörper 101, 112 keinen Stützkörper aufweisen, wie er nachstehend in Verbindung mit den Fig. 9 bis 14 erläutert wird.

Die Fig. 1 und 2 zeigen ein erstes Ausführüngsbeispiel einer bekannten Schultergelenkprothese. Ein Schaft 105 ist in einem Oberarmknochen 103 implantiert, wobei 105 direkt in einem vorbereiteten Knochen bett verankert ist. Bei dem Schaft 105 kann es sich aber ebenso gut um einen mit Knochenzement im Oberarmknochen 103 verankerten Schaft 105 handeln.

In Richtung einer Längsachse 109 für das eigentliche Schultergelenk ist eine Bohrung 116 vorgesehen, die in einer Gegenform 115 für einen konischen Körper 107 endet. Das eigentliche Gelenk wird durch einen mit dem konischen Körper 107 starr verbundenem Lagerkopf 101 und eine Lagerschale 102 gebildet, die ihrerseits mit einer im Schulterknochen 104 verankerten Plattform 106 starr verbunden ist.

Zur Verankerung der Plattform 106 sind parallel zueinander verlaufende Zapfen 114 an der Plattform 106 angebracht, die beispielsweise mit Knochenzement oder durch Presssitz in vorbereiteten Bohrungen des Schulterknochens 104 verankert sind.

Der konische Körper 107 bildet hier eine als Spannabschnitt dienende Kupplung zur Verbindung des in dem Beispiel der Fig. 1 und 2 als Lagerkopf 101 ausgebildeten Lagerkörpers mit dem Schaft 105, der hierzu die als Kupplungsaufnahme dienende Gegenform 115 für den Spannkonus 107 aufweist.

Der konische Körper 107 - und entsprechend die Gegenform 115 - besitzen jeweils einen Querschnitt 110 mit einem Umfang 108, der gemäß Fig. 3 elliptisch ausgebildet ist.

In Fig. 4-sind die angestrebten Verhältnisse bei einem annähernd elliptischen Querschnitt gezeigt. Es sind durch geringfügige Formabweichungen zwischen dem konischen Köper 107 und der Gegenform 115 vier Kontaktpunkte Pvorgesehen, die sich bei intensiver Pressung zu Kontaktflächen erweitern.

Ein radialer Abstand 139 eines Kontaktpunktes P ist derart gewählt, dass eine Normalkraft N in einem Kontaktpunkt P mit ihrer Wirkungslinie in einem relativ großen Abstand 136 an der Längsachse 109 vorbeiführt, um Anteile von einem Drehmoment M als Veränderungen von Normalkräften zu übertragen.

Auf diese Weise wird ein zusätzlich am konischen Körper 107 angreifendes Drehmoment M durch eine Abnahme der Vorspannung bzw. durch eine Erhöhung der Vorspannung N um einen Bruchteil ΔN kompensiert. Der vorgespannte lokale Formschluss ist also entscheidend.

In Fig. 5 ist die gleiche Situation extremer dargestellt. Der konische Köper 107 ist nur noch im Bereich der zu Kontaktflächene erweiterten Kontaktpunkte mit der elliptischen Grundform 115 in Berührung. Die restlichen Flächen sind zurückgesetzt.

Eine weitere Möglichkeit für eine Abänderung des konischen Körpers 107 ist in Fig. 6 dargestellt. Um möglichst große Biegemomente in der Längsachse 109 übertragen zu können, findet die Verspannung in zwei Querschnitten statt, die um einen Mindestabstand 137 auseinander liegen. Dies bedeutet, dass der Konus 107 im mittleren Bereich eine Unterbrechung 138 von diesem Mindestabstand 137 aufweist.

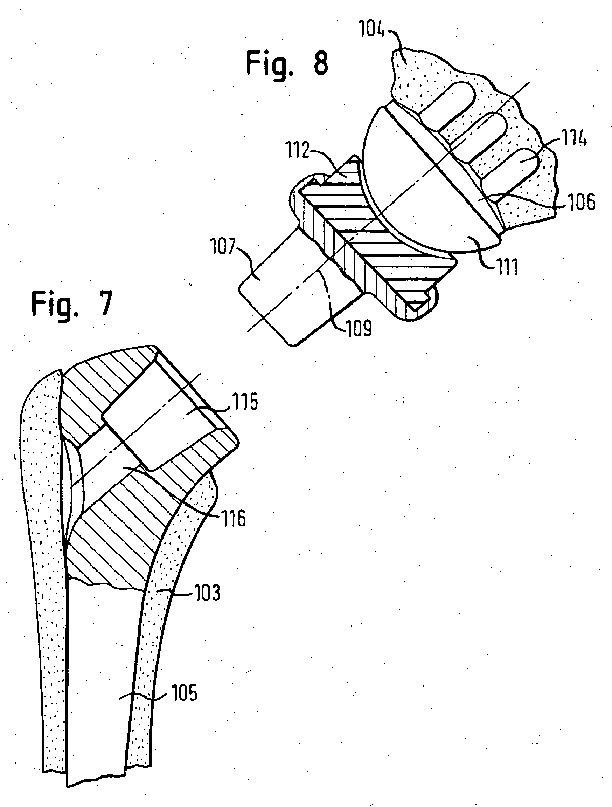

In dem zweiten Ausführungsbeispiel einer bekannten Schultergelenkprothese gemäß Fig. 7 und 8 sind die Funktionen von Kugel- und Lagerschale vertauscht, um den Oberarm um einen Drehpunkt drehen zu lassen. Der mit dem Schaft 105 verbindbare Lagerkörper 112 ist hier in Form einer als Kugelschale ausgebildeten Lagerschale vorgesehen.

Der im Oberarmknochen 103 implantierte Schaft 105 ist mit einer Bohrung 116 sowie einer Gegenform 115 für einen konischen Körper 107 versehen. Jedoch ist der konische Körper zur Aufnahme der Kugelschale 112 verbreitert, die ihrerseits einen Kugelkopf 111 teilweise umschließt. Der Kugelkopf 111 ist durch eine Schnapp- oder Schraubverbindung (nicht gezeigt) auf einer Plattform 106 befestigt, die über Zapfen 114 im Schulterknochen 104 verankert ist. Die Verankerung der Plattform 106 kann ebenso gut über Knochenschrauben und vorstehende Rippen im Schulterknochen 104 erfolgen.

Festigkeitsberechnungen und praktische Versuche haben gezeigt, dass bei einer Anordnung mit einem elliptischen Querschnitt des konischen Körpers 107 und seiner Gegenform 115 eine optimale Ausnutzung des Materials dann stattfindet, wenn die Ellipse in ihrer Ebene so ausgerichtet ist, dass ihre große Achse in einer Projektion gegen lateral als Senkrechte erscheint. Eine derartige Anordnung erlaubt es, bei einer von anterior nach posterior begrenzten Breite vom Schaft 105 ein Maximum an Festigkeit für die konische Klemmverbindung zwischen dem konischen Körper 107 und seiner Gegenform 115 zu erreichen. Dies gilt sowohl für Anordnungen mit einem vollen konischen Körper 107 als auch für einen eine Bohrung aufweisenden konischen Körper, solange der Schaft 105 quer zur Längsachse 109 eine geringere Dicke von posterior nach anterior als in anderen Richtungen aufweist.

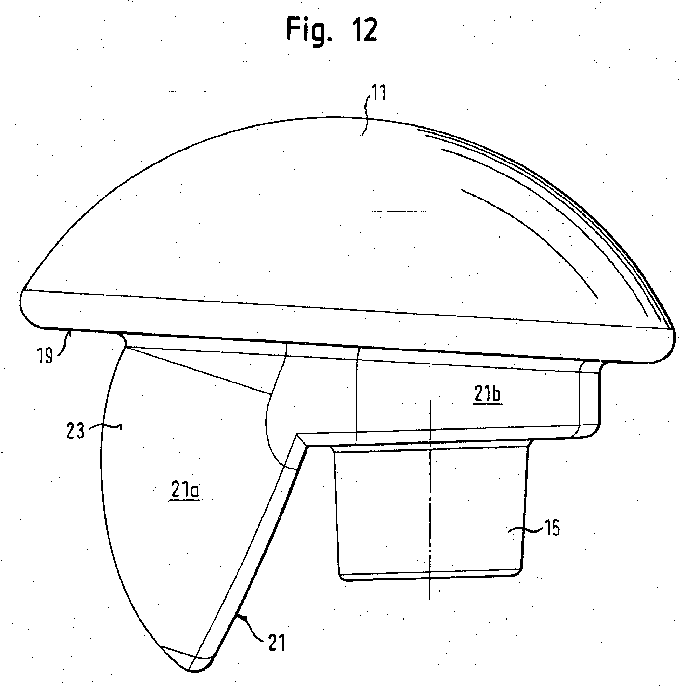

Die Fig. 9 bis 11 zeigen einen Teil einer erfindungsgemäßen Schultergelenkprothese, bei der ein als Lagerkopf ausgebildeter Lagerkörper 11 mit einem Schaft 13 verbunden ist. Den Lagerkörper 11 ohne Schaft 13 zeigt Fig. 12. Der Lagerkörper 11 ist einem als Konus mit elliptischem Querschnitt ausgebildeten Spannabschnitt 15 verbunden, der in eine entsprechend geformte Gegenform des Schaftes 13 einschlagbar ist, um einen festen, wieder lösbaren Spannsitz des Konus 15 im Schaft 13 herzustellen.

Die Orientierung des Lagerkörpers 11 relativ zu dem Konus 15 kann währen der Operation entsprechend den jeweiligen Gegebenheiten innerhalb bestimmter Grenzen beliebig gewählt werden. Hierzu ist der Konus 15 mit einem nicht dargestellten Kupplungsabschnitt versehen, auf welchem der Lagerkörper 11 gelagert ist. Dieser Kupplungsabschnitt ist außerdem derart ausgebildet, dass der Lagerkörper 11 in einer während der Operation gewählten Relativstellung bezüglich des Konus 15 fixiert werden kann.

Diese einstellbare und festsetzbare Koppelung zwischen Konus 15 und Lagerkörper 11 ist nicht Gegenstand der vorliegenden Erfindung, so dass hierauf nicht näher eingegangen wird.

Der Lagerkörper 11 ist mit einem Stützkörper 21 versehen. Der Stützkörper 21 kann einteilig mit dem Lagerkörper 11 ausgebildet oder als separates, mit dem Lagerkörper 11 verbindbares Bauteil vorgesehen sein. Die Verbindung zwischen dem Stützkörper 21 und dem Lagerkörper 11 kann derart fest ausgeführt sein, dass Lagerkörper 11 und Stützkörper 21 eine feste, starre Konfiguration bilden. Alternativ ist es prinzipiell auch möglich, den Stützkörper 21 verstellbar am Lagerkörper 11 anzurbringen, um eine für den jeweiligen Anwendungsfall passende Konfiguration einstellen zu können.

In dem dargestellten Ausführungsbeispiel umfasst der Stützkörper 21 einen schalenartigen Abschnitt 21b und einen kragenartigen Abschnitt 21a, dessen Höhe - d.h. dessen Erstreckung senkrecht zur Unterseite 19 des Lagerkörpers 11 - in die von dem kragenartigen Abschnitt 21a weg weisende Richtung hin abnimmt.

Der Stützkörper 21 dient dazu, den Zwischenraum, der bei mit dem Schaft 13 verbundenem Lagerkörper 11 zwischen der Unterseite 19 des Lagerköpers 11 und dem Schaft 13 vorhanden ist, zu schließen und mit seiner Außenseite eine Stützfläche 23 bereitzustellen, an der sich Knochenfragmente abstützen können, wodurch deren Zusammenwachsen begünstigt wird.

In dem dargestellten Ausführungsbeispiel schließt sich die Stützfläche 23 des Stützkörpers 21 an die Mantelfläche des Schaftes 13 an, die hier im Bereich des Übergangs vom Schaft 13 in den Stützkörper 21 von einem seitlich auf dem Schaft 13 aufsitzenden, korbartigen Stützelement 27 gebildet wird, auf das nachstehend näher eingegangen wird.

Abgesehen von im Stützelement 27 ausgebildeten Durchbrüchen 29 bilden die Stützfläche 23 des Stützkörpers 21 und die im hier interessierenden Übergangsbereich von der Außenseite des Stützelements 27 gebildete Mantelfläche des Schaftes 13 zusammen eine geschlossene, sich an die Unterseite 19 des Lagerkörpers 11 anschließende Fläche. Die konvexe Stützfläche 23 schließt dabei tangential an die Mantelfläche des Schaftes 13 bzw. an die Außenseite des Stützelements 27 an.

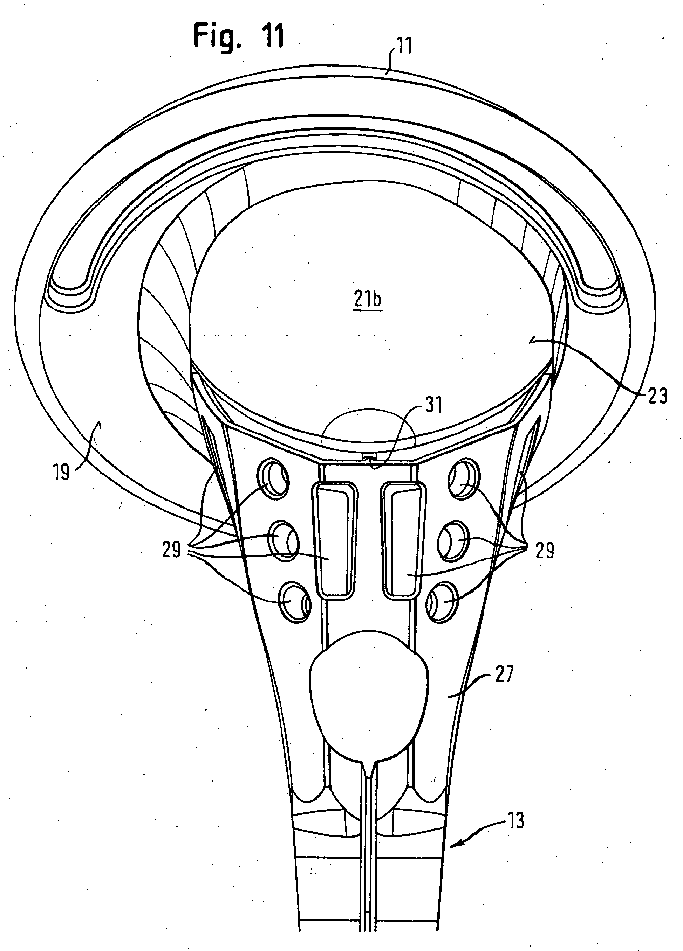

Wie insbesondere Fig. 11 zeigt, ist bei mit dem Schaft 13 verbundenem Lagerkörper 11 zwischen dem Stützelement 27 des Schaftes 13 und dem schalenartigen Abschnitt 21b des Stützkörper 21 eine Aussparung 31 vorhanden, an die zum Lösen des Lagerkörpers 11 von dem Schaft 13 ein Werkzeug angesetzt werden kann.

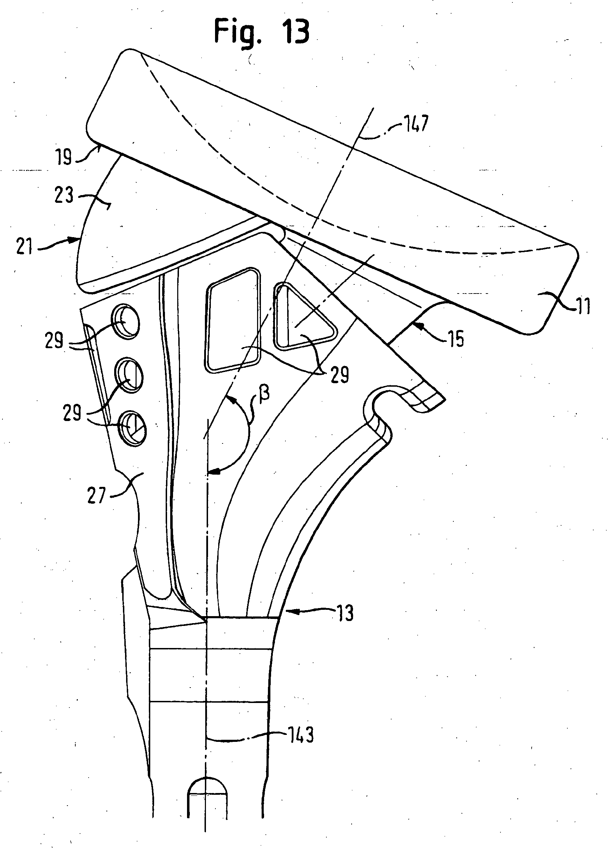

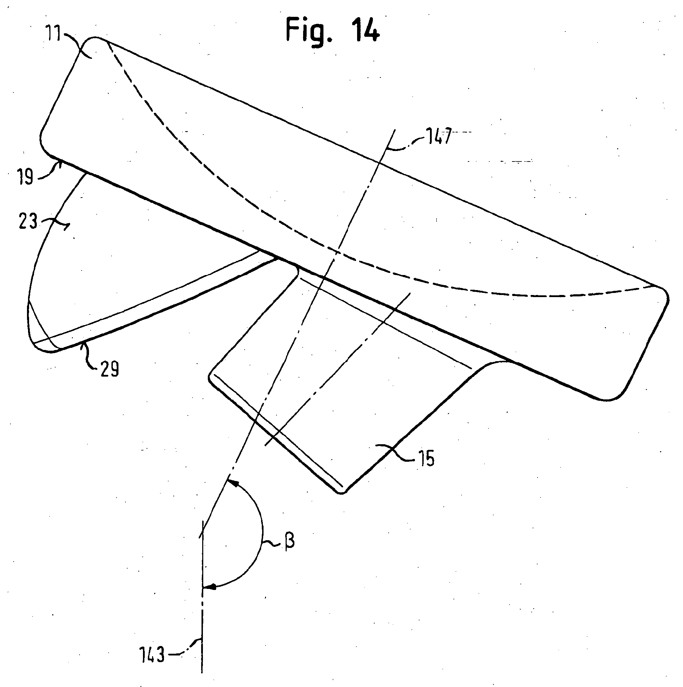

Bei der in den Fig. 13 und 14 dargestellten Ausführungsform der erfindungsgemäßen Schultergelenkprothese ist der Lagerkörper 11 als Kugelschale ausgebildet. Der Stützkörper 21 umfasst hier lediglich einen schalenförmigen Abschnitt, dessen Außenseite die Stützfläche 23 bildet. Ein zusätzlicher kragenförmiger Abschnitt ist in diesem Beispiel nicht vorgesehen, wobei aber auch bei Schultergelenkprothesen mit als Kugelschale ausgebildetem Lagerkörper 11 der Stützkörper 21 grundsätzlich einen zusätzlichen Stützabschnitt z.B. in Kragenform aufweisen könnte. Abgesehen davon gilt auch für dieses Ausführungsbeispiel das in Verbindung mit den vorstehend erläuterten Figuren Gesagte entsprechend.

Die in den Fig. 9, 13 und 14 eingezeichneten Winkel α, β zwischen der Schaftachse 143 und der Kopf- bzw. Schalenachse 147 werden jeweils in Abhängigkeit von den jeweiligen anatomischen Verhältnissen gewählt. In den dargestellten Ausführungsbeispielen gilt α = 130° und β = 155°.

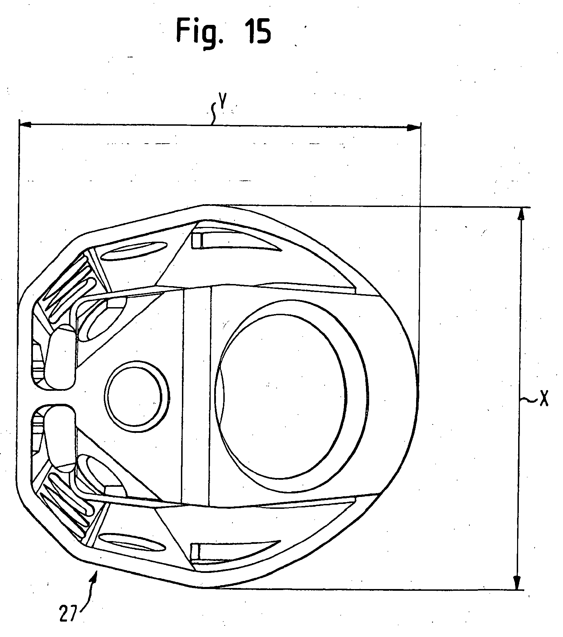

Fig. 15 zeigt das in den Fig. 9 - 11 und 13 dargestellte korb- oder köcherförmige Stützelement 27 in einer Ansicht von oben längs der Schaftachse 143 (vgl. z. B. Fig. 9 und 13), d.h. die Schaftachse 143 verläuft senkrecht zur Zeichenebene in Fig. 15.

Bezogen auf die Orientierung des Stützelementes 27 im implantierten Zustand mit senkrecht zur Transversalebene verlaufender Schaftachse 143 sind der maximale Außendurchmesser in sagittaler Richtung X und der maximale Außendurchmesser in transversaler Richtung Y derart gewählt, dass für alle innerhalb eines Implantatsatzes vorgesehenen Größen des Stützelementes 27 das Verhältnis X/Y im Bereich von 0,85 bis 0,95 liegt.

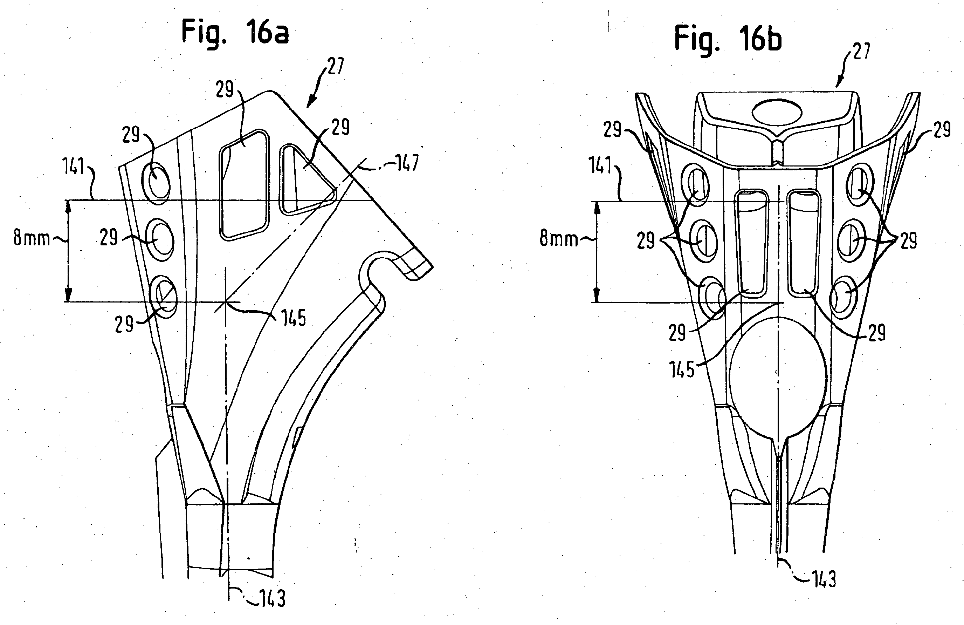

Ferner hat sich als vorteilhaft herausgestellt, die Durchbrüche 29 in dem Stützelement 27 derart vorzusehen, dass die im Folgenden anhand der Fig. 16a und 16b erläuterte Bedingung erfüllt ist.

Die Durchbrüche 29 sind derart ausgebildet und positioniert, dass entlang einer Umfangslinie 141 des Stützelementes 27, die im implantierten Zustand in einer Ebene liegt, welche senkrecht zur Schaftachse 143 und in proximaler Richtung in einem Abstand von 8 mm von dem Schnittpunkt 145 zwischen der Schaftachse 143 und der Kopf- bzw. Schalenachse 147 verläuft, der Anteil der aufgrund der Durchbrüche 29 unterbrochenen Abschnitte der Außenfläche des Stützelementes 27 im Bereich von 35 % bis 45 % liegt.

Versuche haben ergeben, dass hierdurch ein optimaler Kompromiss zwischen Abstützung von Bruchstücken der Knochenfraktur und deren Zusammenwachsen mit durch die Durchbrüche 29 eingebrachten Knochenspänen stattfindet.

### Bezugszeichenliste

- 11: Lagerkörper
- 13: Schaft
- 15: Kupplung, Spannabschnitt, Konus
- 19: Unterseite des Lagerkörpers
- 21: Stützkörper
- 21a: kragenförmiger Abschnitt des Stützkörpers
- 21b: schalenförmiger Abschnitt des Stützkörpers
- 23: Stützfläche
- 27: Stützelement,
- 29: Durchbruch
- 31: Aussparung

- 101: Lagerkopf
- 102: Lagerschale 103 Oberarmknochen
- 104: Schulterknochen
- 105: Schaft
- 106: Plattform
- 107: konischer Körper
- 108: Umfang
- 109: Längsachse
- 110: Querschnitt
- 111: Kugelkopf
- 112: Kugelschale
- 114: Zapfen
- 115: Gegenform
- 116: Bohrung
- 136: Abstand
- 137: Mindestabstand
- 138: Unterbrechung
- 139: radialer Abstand
- 141: Umfangslinie
- 143: Schaftachse 145 Schnittpunkt
- 147: Kopfachse, Schalenachse

## Patentansprüche

1. Schultergelenkprothese mit zwei zusammenwirkenden Lagerkörpern (11), einem Schaft (13) und einer Kupplung (15) zum Verbinden des Schaftes (13) mit einem der Lagerkörper (11),
wobei sich an die Unterseite (19) des mit dem Schaft (13) verbindbaren Lagerkörpers (11) ein Stützkörper (21) anschließt, der im mit dem Schaft (13) verbundenen Zustand mit einer äußeren Stützfläche (23) eine zwischen dem Lagerkörper (11) und dem Schaft (13) vorhandene Lücke zumindest teilweise derart ausfüllt, dass das Zusammenwachsen von Knochenfragmenten begünstigt wird,
**dadurch gekennzeichnet,**
**dass** der Schaft (13) mit einem Stützelement (27) versehen ist, das seitlich auf dem Schaft (13) aufsitzt und im Bereich des Übergangs vom Schaft (13) in den Stützkörper (21) mit seiner Außenseite die in die Stützfläche (23) des Stützkörpers (21) übergehende Mantelfläche des Schaftes (13) bildet.

2. Schultergelenkprothese nach Anspruch 1,
**dadurch gekennzeichnet,**
**dass** die Kupplung zur Verbindung mit dem Schaft (13) einen Spannabschnitt (15) umfasst, mit dem durch Einbringen in eine Kupplungsaufnahme des Schaftes (13) ein fester, insbesondere wieder lösbarer Spannsitz der Kupplung (15) im Schaft (13) herstellbar ist.

3. Schultergelenkprothese nach Anspruch 2,
**dadurch gekennzeichnet,**
**dass** der Spannabschnitt (15) sich konusartig verjüngt und in eine entsprechend geformte, als Kupplungsaufnahme dienende Gegenform des Schaftes (13) einbringbar ist.

4. Schultergelenkprothese nach Anspruch 2 oder 3,
**dadurch gekennzeichnet,**
**dass** der Spannabschnitts (15) im mit dem Schaft (13) verbundenen Zustand einen zu einer Längsachse drehfesten und durch seine Konizität verkeilten Formschluss mit der Gegenform bildet.

5. Schultergelenkprothese nach einem der Ansprüche 2 bis 4,
**dadurch gekennzeichnet,**
**dass** der Spannabschnitt (15) einen von einer Kreisforin abweichenden Querschnitt aufweist.

6. Schultergelenkprothese nach einem der Ansprüche 2 bis 5,
**dadurch gekennzeichnet,**
**dass** der Spannabschnitt (15) einen elliptischen Querschnitt aufweist.

7. Schultergelenkprothese nach Anspruch 6,
**dadurch gekennzeichnet,**
**dass** der elliptische Querschnitt des Spannabschnitts (15) und der Gegenform des Schaftes (13) derart in seiner Ebene ausgerichtet ist, dass die große Achse der Ellipse in einer Projektion gegen lateral als Senkrechte erscheint.

8. Schultergelenkprothese nach Anspruch 6 oder 7,
**dadurch gekennzeichnet,**
**dass** der Schaft (13) in der Ebene des elliptischen Querschnitts den Umriss eines mit abgerundeten Ecken versehenen Rechtecks aufweist, dessen langen Seiten parallel zu der großen Achse der Ellipse verlaufen.

9. Schultergelenkprothese nach einem der vorhergehenden Ansprüche,
**dadurch gekennzeichnet,**
**dass** die Stützfläche (23) des Stützkörpers (21) zumindest bereichsweise konvex ist.

10. Schultergelenkprothese nach einem der vorhergehenden Ansprüche,
**dadurch gekennzeichnet,**
**dass** die Mantelfläche des Schaftes (13) und die Stützfläche (23) des Stützkörpers (21) zusammen eine zumindest im Wesentlichen geschlossene, sich an die Unterseite (19) des Lagerkörpers (11) anschließende Fläche bilden.

11. Schultergelenkprothese nach einem der vorhergehenden Ansprüche,
**dadurch gekennzeichnet,**
**dass** die Stützfläche (23) des Stützkörpers (21) zumindest näherungsweise tangential an die Mantelfläche des Schaftes (13) anschließt.

12. Schultergelenkprothese nach einem der vorhergehenden Ansprüche,
**dadurch gekennzeichnet,**
**dass** die Stützfläche (23) des Stützkörpers (21) strukturiert ist.

13. Schultergelenkprothese nach einem der vorhergehenden Ansprüche,
**dadurch gekennzeichnet,**
**dass** die Stützfläche (23) des Stützkörpers (21) durchbrechungsfrei ausgebildet ist.

14. Schultergelenkprothese nach einem der vorhergehenden Ansprüche,
**dadurch gekennzeichnet,**
**dass** der Stützkörper (21) auswechselbar mit dem Lagerkörper (11) verbunden ist.

15. Schultergelenkprothese nach einem der vorhergehenden Ansprüche,
**dadurch gekennzeichnet,**
**dass** bei mit dem Schaft (13) verbundenem Lagerkörper (11) zwischen dem Schaft (13) und dem Stützkörper (21) wenigstens eine Aussparung (31) vorhanden ist, an die ein Werkzeug zum Lösen des Lagerkörpers (11) von dem Schaft (13) ansetzbar ist.

16. Schultergelenkprothese nach einem der vorhergehenden Ansprüche,
**dadurch gekennzeichnet,**
**dass** das Stützelement (27) korb- oder köcherartig ausgebildet ist.

17. Schultergelenkprothese nach einem der vorhergehenden Ansprüche,
**dadurch gekennzeichnet,**
**dass** das Stützelement (27) fest mit dem Schaft (13) verbunden, insbesondere mit dem Schaft verschweißt ist.

18. Schultergelenkprothese nach einem der Ansprüche 1 bis 16,
**dadurch gekennzeichnet,**
**dass** das Stützelement (27) einteilig mit dem Schaft (13) ausgebildet ist.

19. Schultergelenkprothese nach einem der vorhergehenden Ansprüche,
**dadurch gekennzeichnet;**
**dass** das Stützelement (27) mit Durchbrüchen (29) versehen ist.

20. Schultergelenkprothese nach Anspruch 19,
**dadurch gekennzeichnet,**
**dass** entlang einer Umfangslinie (141) des Stützelementes (27), die im implantierten Zustand in einer senkrecht zur Schaftachse (143) und in proximaler Richtung in einem Abstand von 8 mm von dem Schnittpunkt (145) zwischen der Schaftachse (143) und der Kopf-bzw. Schalenachse (147) verlaufenden Ebene liegt, der Anteil der aufgrund der Durchbrüche (29) unterbrochenen Abschnitte der Außenfläche des Stützelementes (27) im Bereich von 35 % bis 45 % liegt.

21. Schultergelenkprothese nach einem der vorhergehenden Ansprüche,
**dadurch gekennzeichnet,**
**dass** bezogen auf die Orientierung des Stützelementes (27) im implantierten Zustand mit senkrecht zur Transversalebene verlaufender Schaftachse (143) das Verhältnis X/Y aus maximalem Außendurchmesser des Stützelementes (27) in sagittaler Richtung X und maximalem Außendurchmesser des Stützelementes (27) in transversaler Richtung Y im Bereich von 0,85 bis 0,95 liegt.

## Claims

1. A shoulder joint prosthesis comprising two cooperating bearing bodies (11), a shaft (13) and a coupling (15) for the connection of the shaft (13) to one of the bearing bodies (11),
wherein a support member (21) adjoins the lower side (19) of the bearing body (11) connectable to the shaft (13), an outer support surface (23) of said support member (21) at least partly filling a gap present between the bearing body (11) and the shaft (13) in the state connected to the shaft (13) such that the growing together of bone fragments is promoted,
**characterized in that**
the shaft (13) is provided with a support element (27) which is mounted laterally at the shaft (13) and whose outer side forms the jacket surface of the shaft (13) merging into the support surface (23) of the support member (21) in the region of the transition from the shaft (13) into the support member (21).

2. A shoulder joint prosthesis in accordance with claim 1,
**characterized in that**
the coupling for the connection to the shaft (13) includes a clamping section (15) with which a firm clamping seat of the coupling (15) in the shaft (13) can be established by introduction into a coupling mount of the shaft (13), in particular a clamping seat which can be released again.

3. A shoulder joint prosthesis in accordance with claim 2,
**characterized in that**
the clamping section (15) tapers like a cone and can be introduced into a correspondingly shaped counter shape of the shaft (13), which serves as a coupling mount.

4. A shoulder joint prosthesis in accordance with claim 2 or claim 3,
**characterized in that**
the clamping section (15) forms a shape matched connection with the counter shape which is rotationally fixed with respect to a longitudinal axis and is wedged due to its conical shape in the state connected to the shaft (13).

5. A shoulder joint prosthesis in accordance with any one of claims 2 to 4,
**characterized in that**
the clamping section (15) has a cross-section deviating from a circular shape.

6. A shoulder joint prosthesis in accordance with any one of claims 2 to 5,
**characterized in that**
the clamping section (15) has an elliptical cross-section.

7. A shoulder joint prosthesis in accordance with claim 6,
**characterized in that**
the elliptical cross-section of the clamping section (15) and of the counter shape of the shaft (13) is aligned in its plane such that the major axis of the ellipse appears as a perpendicular in a projection toward lateral.

8. A shoulder joint prosthesis in accordance with claim 6 or claim 7,
**characterized in that**
the shaft (13) has, in the plane of the elliptical cross-section, the outline of a rectangle provided with rounded corners whose long sides extend in parallel with the major axis of the ellipse.

9. A shoulder joint prosthesis in accordance with any one of the preceding claims,
**characterized in that**
the support surface (23) of the support member (21) is at least regionally convex.

10. A shoulder joint prosthesis in accordance with any one of the preceding claims,
**characterized in that**
the jacket surface of the shaft (13) and the support surface (23) of the support member (21) together form an at least substantially closed surface which adjoins the lower side (19) of the bearing body (11).

11. A shoulder joint prosthesis in accordance with any one of the preceding claims,
**characterized in that**
the support surface (23) of the support member (21) at least approximately tangentially adjoins the jacket surface of the shaft (13).

12. A shoulder joint prosthesis in accordance with any one of the preceding claims,
**characterized in that**
the support surface (23) of the support member (21) is structured.

13. A shoulder joint prosthesis in accordance with any one of the preceding claims,
**characterized in that**
the support surface (23) of the support member (21) is made free of apertures.

14. A shoulder joint prosthesis in accordance with any one of the preceding claims,
**characterized in that**
the support member (21) is replaceably connected to the bearing body (11).

15. A shoulder joint prosthesis in accordance with any one of the preceding claims,
**characterized in that**,
when the bearing body (11) is connected to the shaft (13), at least one recess (31) is present between the shaft (13) and the support member (21) at which a tool can be positioned to release the bearing body (11) from the shaft (13).

16. A shoulder joint prosthesis in accordance with any one of the preceding claims,
**characterized in that**
the support element (27) is of basket-like or quiver-like shape.

17. A shoulder joint prosthesis in accordance with any one of the preceding claims,
**characterized in that**
the support element (27) is fixedly connected to the shaft (13) and is in particular welded to the shaft.

18. A shoulder joint prosthesis in accordance with any one of the claims 1 to 16,
**characterized in that**
the support element (27) is made in one piece with the shaft (13).

19. A shoulder joint prosthesis in accordance with any one of the preceding claims
**characterized in that**
the support element (27) is provided with apertures (29).

20. A shoulder joint prosthesis in accordance with claim 19,
**characterized in that**
the proportion of the sections of the outer surface of the support element (27), which is interrupted due to the apertures (29), lies in the range from 35% to 45% along a peripheral line (141) of the support element (27) which lies in the implanted state in a plane extending perpendicular to the shaft axis (143) and, in the proximal direction, at a spacing of 8 mm from the point of intersection (145) between the shaft axis (143) and the head axis or shell axis (147).

21. A shoulder joint prosthesis in accordance with any one of the preceding claims,
**characterized in that**,
with respect to the orientation of the support element (27) in the implanted state with the shaft axis (143) extending perpendicular to the transverse plane, the ratio X/Y of the maximum outer diameter of the support element (27) in the sagittal direction X and the maximum outer diameter of the support element (27) in the transverse direction Y lies in the range from 0.85 to 0.95.

## Revendications

1. Prothèse de l'articulation de l'épaule, comportant deux corps formant palier (11), une tige (13) et un accouplement (15) pour relier la tige (13) à l'un des corps formant palier (11), dans laquelle à la face inférieure (19) du corps formant palier (11) susceptible d'être relié à la tige (13) se raccorde un corps de soutien (21) qui, dans l'état relié avec la tige (13), remplit au moins partiellement avec une surface de soutien (23) extérieure une lacune se trouvant entre le corps formant palier (11) et la tige (13), de telle sorte que la croissance commune de fragments osseux est favorisée, **caractérisée en ce que** la tige (13) est pourvue d'un élément de soutien (27) qui repose latéralement sur la tige (13) et qui, dans la zone de la transition depuis la tige (13) jusque dans le corps de soutien (21), forme avec sa face extérieure la surface enveloppe de la tige (13) qui passe avec sa face extérieure dans la surface d'appui (23) du corps de soutien (21).

2. Prothèse de l'articulation de l'épaule selon la revendication 1, **caractérisée en ce que** l'accouplement pour la liaison avec la tige (13) comprend une portion de serrage (15) avec laquelle, par introduction dans un logement d'accouplement de la tige (13), une assise par serrage détachable de l'accouplement (15) dans la tige (13) peut être réalisée.

3. Prothèse de l'articulation de l'épaule selon la revendication 2, **caractérisée en ce que** la portion de serrage (15) se rétrécit de manière conique et peut être introduite dans une forme antagoniste de forme correspondante de la tige (13) qui sert de logement d'accouplement.

4. Prothèse de l'articulation de l'épaule selon la revendication 2 ou 3, **caractérisée en ce que** la portion de serrage (15), dans l'état relié avec la tige (13), forme avec la forme antagoniste un engagement par coopération de formes solidaire en rotation à un axe longitudinal et calé grâce à sa conicité.

5. Prothèse de l'articulation de l'épaule selon l'une des revendications 2 à 4, **caractérisée en ce que** la portion de serrage (15) présente une section transversale différente d'une forme circulaire.

6. Prothèse de l'articulation de l'épaule selon l'une des revendications 2 à 5, **caractérisée en ce que** la portion de serrage (15) présente une section transversale elliptique.

7. Prothèse de l'articulation de l'épaule selon la revendication 6, **caractérisée en ce que** la section transversale elliptique de la portion de serrage (15) et de la forme antagoniste de la tige (13) est orientée de telle sorte dans son plan que le grand axe de l'ellipse apparaît comme une verticale dans une projection latérale.

8. Prothèse de l'articulation de l'épaule selon la revendication 6 ou 7, **caractérisée en ce que** la tige (13) présente dans le plan de la section transversale elliptique les contours d'un rectangle pourvu de coins arrondis, dont les grands côtés s'étendent parallèlement au grand axe de l'ellipse.

9. Prothèse de l'articulation de l'épaule selon l'une des revendications précédentes, **caractérisée en ce que** la surface d'appui (23) du corps d'appui (21) est convexe au moins sur certaines zones.

10. Prothèse de l'articulation de l'épaule selon l'une des revendications précédentes, **caractérisée en ce que** la surface enveloppe de la tige (13) et la surface d'appui (23) du corps de soutien (21) forment ensemble une surface au moins sensiblement fermée qui se raccorde à la face inférieure (19) du corps formant palier (11).

11. Prothèse de l'articulation de l'épaule selon l'une des revendications précédentes, **caractérisée en ce que** la surface d'appui (23) du corps de soutien (21) se raccorde de manière au moins approximativement tangentielle à la surface enveloppe de la tige (13).

12. Prothèse de l'articulation de l'épaule selon l'une des revendications précédentes, **caractérisée en ce que** la surface d'appui (23) du corps de soutien (21) est structurée.

13. Prothèse de l'articulation de l'épaule selon l'une des revendications précédentes, **caractérisée en ce que** la surface d'appui (23) du corps de soutien (21) est réalisée sans percée.

14. Prothèse de l'articulation de l'épaule selon l'une des revendications précédentes, **caractérisée en ce que** le corps de soutien (21) est relié de manière interchangeable avec le corps formant palier (11).

15. Prothèse de l'articulation de l'épaule selon l'une des revendications précédentes, **caractérisée en ce que** dans le cas où le corps formant palier (11) est relié à la tige (13), il est prévu entre la tige (13) et le corps formant palier (21) au moins un évidement (31) sur lequel un outil peut être monté pour détacher le corps formant palier (11) vis-à-vis de la tige (13).

16. Prothèse de l'articulation de l'épaule selon l'une des revendications précédentes, **caractérisée en ce que** l'élément de soutien (27) est réalisé en forme de corbeille ou de fourreau.

17. Prothèse de l'articulation de l'épaule selon l'une des revendications précédentes, **caractérisée en ce que** l'élément de soutien (27) est relié de manière solidaire à la tige (13), en particulier soudé à la tige.

18. Prothèse de l'articulation de l'épaule selon l'une des revendications 1 à 16, **caractérisée en ce que** l'élément de soutien (27) est réalisé d'un seul tenant avec la tige (13).

19. Prothèse de l'articulation de l'épaule selon l'une des revendications précédentes, **caractérisée en ce que** l'élément de soutien (27) est pourvu de percées (29).

20. Prothèse de l'articulation de l'épaule selon la revendication 19, **caractérisée en ce que** le long d'une ligne périphérique (141) de l'élément de soutien (27) qui, à l'état implanté, se trouve dans un plan s'étendant perpendiculairement à l'axe de la tige (143) et en direction proximale à une distance de 8 mm par rapport au point d'intersection (145) entre l'axe de la tige (143) et l'axe de tête ou de coque (147), la part des tronçons de la surface extérieure de l'élément de soutien (27), qui sont interrompus en raison des percées (29), est dans la plage de 35% à 45%.

21. Prothèse de l'articulation de l'épaule selon l'une des revendications précédentes, **caractérisée en ce que** par rapport à l'orientation de l'élément de soutien (27) à l'état implanté avec axe de tige (143) s'étendant perpendiculairement au plan transversal, le rapport X/Y du diamètre extérieur maximum de l'élément de soutien (27) en direction sagittale X sur le diamètre extérieur maximum de l'élément de soutien (27) en direction transversale Y est dans la plage de 0,85 à 0,95.
